# EUROPEAN PATENT SPECIFICATION

(11) **EP 2 965 744 B1**
(45) Date of publication and mention of the grant of the patent: **15.03.2017**
(21) Application number: 15176194.7
(22) Date of filing: 10.07.2015
(51) Int. Cl.: A61K 8/26, A45D 31/00, A61Q 3/02, A61K 8/02

(54) **ALUMINUM-CONTAINING HIGH GLOSS COATING FOR NATURAL AND ARTIFICIAL FINGERNAILS**
ALUMINIUMHALTIGE HOCHGLANZBESCHICHTUNG FÜR NATÜRLICHE UND KÜNSTLICHE FINGERNÄGEL
REVÊTEMENT À BRILLANCE ÉLEVÉE CONTENANT DE L'ALUMINIUM POUR ONGLES NATURELS ET ARTIFICIELS

(30) Priority: 11.07.2014 US 201462023364 P
(43) Date of publication of application: 13.01.2016
(73) Proprietor: Mycone Dental Supply Company Inc., Cherry Hill, NJ 08002 (US)
(72) Inventor: GOUSE, Kendra, Philadelphia, PA Pennsylvania 19122 (US)
(74) Representative: Carpintero Lopez, Francisco

(56) References cited:
- WO-A1-2013/155094
- WO-A2-02/03913
- KR-B1- 101 342 492
- US-A1- 2004 241 423
- US-A1- 2008 131 383

## Description

### BACKGROUND OF THE INVENTION

This invention relates to the field of metallic-appearing coatings useful for cosmetic adornment of natural fingernails and toenails, artificial fingernails and toenails, and artificial nail extensions.

More particularly the invention relates to the field of actinic radiation cured coatings for such nails.

The use of radiation-curable gels in formation of nail enhancements or artificial nails has been an important part of the cosmetic industry since it was first introduced. US Pat. 4,682,612, describing the use of actinic radiation-curable compositions suitable for preparation of artificial nails, is representative of this technology.

Ultra-violet radiation (UV) is the most conventional form of radiation used to cure gels in this art. Professional nail technicians most typically apply UV curable gels which are usually composed of acrylic or methacrylic monomers and oligomers in a gel-like state that requires curing under a UV lamp. Typically these can be broad wavelength lamps or narrow wavelength lamps based on Light Emitting Diodes (LED). Visible light curing can also be used. Such nail finishes can be applied directly to natural fingernails or toenails, or alternatively can be applied to nail extensions bonded to fingernails.

A wide variety of colors and finishes are used by the nail technicians to achieve a desired appearance of the natural or artificial nail. Various attempts have been described in the art to impart a metallic finish to nails. See, for example, Ilekti, et al., US Pat. Pub. 2009/0126316, which describes a flexible article with an optical relief comprising an adhesive layer, an organic film, and a material with an optical relief, or olfactory effect, wherein the organic film may contain various metallic particles; US 6,565,835 which describes a nail enamel composition of non-toxic components for forming a film over natural or synthetic human nails, comprising a film forming component, a solvent, and aluminum platelets, said film having a haze value greater than 932 HU. and US2004/0241423 discloses a nail vanish with a mirror-effect wherein the varnish consists of two layers, namely a base coat comprising aluminum platelets and film forming polymer (cellulose acetate butyrate) and a top coat based on film former (cellulose acetate butyrate).

The prior systems for coating such nails do not provide acceptable mirror-like metallic finishes for various reasons.

It is an object of the present invention to provide mirror-like metallic finish systems for such nails.

### SUMMARY OF THE INVENTION

This object and others which will become apparent from the following description are achieved by the present invention which comprises in one aspect a multi-layer cosmetic laminate for decorating natural or artificial human nails comprised of at least three layers, a UV cured coating as the first layer, a layer comprising aluminum platelets as the second layer, and another UV cured coating layer as the third layer, the second layer which comprises aluminum platelets being substantially free of film former.

Another aspect of the invention comprises a method of decorating natural or artificial human nails comprising
a) applying a first layer comprising a UV curable gel coating composition comprising one or more ethylenically unsaturated monomers, one or more ethylenically unsaturated oligomers, or mixtures of said ethylenically unsaturated monomer or monomers and said ethylenically unsaturated oligomer or oligomers, and a photoinitiator;
b) curing the first layer under UV radiation;
c) applying a second layer comprising aluminum platelets which is substantially free of film former;
d) applying a third layer comprising the UV curable gel coating composition; and
e) curing the third layer under UV radiation.

The laminate is not limited to three layers in that additional layers can be provided before, after, or between two of the three layers. The additional layers can be metallic, adhesive, UV cured gels, or enamel.

Very reflective composites are provided, having a haze value in most cases substantially greater than 950 HU.

As is conventional, in most embodiments UV cured coating layers are each formed by curing under UV radiation a composition comprising one or more ethylenically unsaturated monomers, one or more ethylenically unsaturated oligomers, or mixtures of said ethylenically unsaturated monomer or monomers and said ethylenically unsaturated oligomer or oligomers, and a photoinitiator. It is also possible for said coatings to contain only a monomer or monomers or only an oligomer or oligomers.

Preferably the monomers and/or oligomers are selected from mono-, di-, tri-, and tetra-functional ethylenically unsaturated monomers and/or oligomers and are most preferably at least in part (meth)acrylic such as aliphatic polyester or polyether based urethane diacrylate oligomers.

According to this invention the layer comprising aluminum platelets is formed subsequent to curing the first base coat layer by coating the cured first base coat layer with a suspension of aluminum platelets in solvent and then evaporating the solvent to form a dry coating of aluminum.

According to this invention the uncured layer of base coat which may result during the curing to form the base coat layer, known in the art as the tacky layer, is removed using a suitable solvent, such as isopropanol, prior to application of the suspension of aluminum platelets.

According to this invention the uncured layer of top coat which may result during the curing to form the top coat layer, known in the art as the tacky layer, is removed using a suitable solvent, such as isopropanol after curing.

The preferred solvents for the aluminum platelet suspension are those most widely used in the enamel nail coating art, namely ethyl acetate, butyl acetate, acetone, isopropanol and mixtures thereof

Preferably the third coat and any other coats over the aluminum platelet layer are free of pigment so that the metal appearance of the aluminum layer is clearly visible.

The suspension of aluminum platelets can contain a leafing agent, for example a long chain phosphoric acid ester.

The aluminum platelets of the second layer can be made by a variety of methods, for example one which comprises physical vapor deposition. Aluminum platelets are available, for example, from Schlenk under the Decomet® trademark, Eckart Division of Altana under the Metalure® trademark and Silberline under the Starbrite® trademark. One preferred brand of aluminum platelets is the Decomet® brand.

The suspension of aluminum platelets may contain a viscosity modifier which is not a film former.

In some embodiments the aluminum platelets are multi-layered effect pigments created by physical vapor deposition having an aluminum core with MgF2 layers deposited thereon and a grating with a defined spacing between grating structures embossed on these low refracting layers which produces a prismatic rainbow-like diffraction effect when observed under visible light.

In some embodiments, a fourth layer is coated on top of the three layer laminate.

### DETAILED DESCRIPTION

Examples of urethane(meth)acrylates, useful in the present invention have at least one, preferably two or more acryloyl, or methacryloyl groups and a urethane group. Examples include urethanes based on aliphatic, aromatic, polyester, and polyether polyols and aliphatic, aromatic, polyester, and polyether diisocyanates capped with (meth)acrylate endgroups and urethane (meth)acrylates made from reaction of aliphatic or aromatic isocyanates with hydroxyl containing (meth)acrylic monomers or oligomers. Other oligomers useful in the invention include epoxy (meth) acrylates and epoxy urethane (meth)acrylates having at least one, preferably two or more two or more acryloyl or methacryloyl groups and, optionally a urethane group. Examples include epoxy (meth)acrylates based on aliphatic or aromatic epoxy prepolymers capped with a urethane group (meth)acrylate end group. An aliphatic or aromatic urethane spacer can be optionally inserted between the epoxy and the (meth)acrylate end group(s). (Meth)acrylated polyester oligomers, useful in the present invention have at least one, preferably two or more acryloyl or methacryloyl groups and a polyester core. (Meth)acrylated acrylate oligomers, useful in the present invention, have at least one, preferably two or more acryloyl or methacryloyl groups and a polyacrylic core. (Meth)acrylated polyether oligomers useful in the invention have at least one, preferably two or more acryloyl or methacryloyl groups and a polyether core. (Meth)acrylated cellulose oligomers useful in the invention have at least one, preferably two or more acryloyl groups and a cellulose core. Cellulose based urethanes and maleates containing acryloly, methacryaloyl, styrenic or maleate groups may also be used. Butadiene oligomers have at least one, preferably two or more acryloyl or methacyloyl groups and optionally at least one urethane functionality. (Meth)acrylated styrene/maleic oligomers have at least one, preferably two or more (meth)acryloyl groups attached to a styrene/maleic anhydride core. These materials can be made by methods well known in the art and are available from several suppliers including Bayer, BASF, Dymax, Allnex, DSM Neoresins, Eternal Chemical Company, IGM Resins, Rahn AG, Sartomer Division of Arkema, Esstech Resins, Miwon, Double Bond Chemical, Kowa, Soltech and SI Group. Mixtures of these oligomers may also be used.

In addition to the above-described (meth)acrylate- based polymerizable materials, other polymerizable monomers, oligomers or polymers of monomers which contain at least one free radical polymerizable group in the molecule may be used without any limitations in the curable gel. Typical examples include esters of acyrlic and methacrylic acid, herein termed (meth)acrylic ester. Specific but not limiting examples of mono (meth)acryloyl esters include methyl (meth)acrylate, ethyl (meth)acrylate, hydroxypropyl (meth)acrylate, butyl (meth)acrylates, hydroxy ethyl (meth)acrylates, butoxyethyl (meth)acrylate, diethylaminoethyl (meth)acrylate, 2-ethylhexyl (meth)acrylate, ethoxyethyl (meth)acrylate, t-butyl aminoethyl (meth)acrylate, methoxyethylene glycol (meth)acrylate, phosphoethyl (meth)acrylate, methoxy propyl (meth)acrylate, methoxy polyethylene glycol(meth)acrylate, phenoxyethylene glycol (meth)acrylate, phenoxypolyethylene glycol (meth)acrylate, 2-hydroxy-3-phenoxypropyl (meth)acrylate, 2-(meth)acryloxyethylsuccinic acid, 2-(meth)acryloylethylphthalic acid, 2-(meth)acryloyloxypropylphthalic acid, stearyl (meth)acrylate, isobornyl (meth)acrylate, 3-chloro-2-hydroxypropyl (meth)acrylates, tetrahydrofufuryl (meth)acrylate, methacryloyloxyethyl trimelilitc anhydride, (meth)acrylamides and allyl monomers. Specific but not limiting examples of difunctional (meth)acryloyl esters include 1,4 butane diol di(meth)acrylate, 1,6 hexananediol di(meth)acrylate, alkoxylated hexane diol di(meth)acrylate, 1,9 nonanediol di(meth)acrylate, 1,10 decanediol di(meth)acrylate, neopentyl glycol di(meth)acrylate, alkoxylated neopentyl glycol di(meth)acrylate, 2-methyl-1,8-octane diol di(meth)acrylate, cyclohaxane dimethanol di(meth)acrylate, glycerin di(meth)acrylate, ethylene glycol di(meth)acrylate, triethyleneglycol di(meth)acrylate, polyethylene glycol di(meth)acrylate, propylene glycol di(meth)acrylate, polypropylene glycol di(meth)acrylate, ethoxylated propylene glycol di(meth)acrylate, ethoxylated polypropylene glycol di(meth)acrylate, polyethoxypropoxy di(meth)acrylate, ethoxylated bisphenol A di(meth)acrylate, propoxylated bisphenol A di(meth)acrylate, propoxylated ethoxylated bisphenol A di(meth)acrylate, bisphenol A glycidyl methacrylate, tricyclodecanedimethanol di(meth)acrylate, glycerin di(meth)acrylate, ethoxylated glycerin di(meth)acrylate, bis acrylamides, bis allyl ethers and allyl (meth)acrylates. Examples of tri and or higher (meth)acryloyl esters include trimethylol propane tri(meth)acrylate, ethoxylated glycerin tri(meth)acrylate, ethoxylated trimethylolpropane tri(meth)acrylate, ditrimethylol propane tetra(meth)acrylate, pentaerythritol tr-ir(meth)acrylate, pentaerythritol tetra(meth)acrylate, propoxylated pentaerythritol tetra(meth)acrylate, ethoxylated pentaerythritol tetra(meth)acrylate, dipentaerythritol hexa(meth)acrylate, dipentaerythritol penta(meth)acrylate and ethoxylated iscyanuric acid tri(meth)acrylates. Monomers containing acid groups may also be used including (meth)acrylic acid, bis(gyceryl dimethacrylate) pyromellitate, pyromellitic dimethacrylate, methacryloyloxyethyl phthalate, methacryloyloxyehtyl maleate, 2 hydroxyethyl methacrylate/succinate, 1,3 glyceryl dimethacylate maleate adduct, and 1,3 glyceryl dimethacrylate succinate adduct. Partially aminated monomers and oligomers may also be used. These are prepared by reaction of amines, preferably secondary amines, with some of the (meth)acryloyl groups of the multifunctional monomers or oligomers.

A compound having at least one free radical polymerizable group includes not only a single component but also a mixture of polymerizable monomers. Thus combinations of two or more materials containing free radical polymerizable groups may be used.

The gels also contain a photoinitiator. Examples of these include benzyl ketones, monomeric hydroxyl ketones, polymeric hydroxyl ketones, alpha-amino ketones, acyl phosphine oxides, phosphinates, metallocenes, benzophenone, benzophenone derivatives, and the like. Specific examples include 1-hydroxy-cyclohexylphenylketone,2,2-dimethoxy 2-phenyl acetephenone, 2-hydroxy-2-methyl-1-phenyl-1-propanone, alpha, alpha-dimethoxy alphaphenyl acetophenone, benzophenone, 2-benzyl-2-(dimethylamino)-1-(4-(4-morphorlinyl)phenyl)-1-butanone, 2-methyl-1-(4-methylthio)phenyl-2-(4-morphorlinyl)-1-propanone, diphenyl-(2,4,6-trimethylbenzoyl) phosphine oxide, phenyl bis(2,4,6-trimethylbenzoyl) phosphine oxide, benzyl-dimethylketal, isopropylthioxanthone, ethyl (2,4,6-trimethyl benzoyl) phenyl phosphinate and phenyl (2,4,6-trimethyl benzoyl) phenyl phosphinate, methyl benzoyl formate, and mixtures thereof.

Photo accelerators such as aliphatic or aromatic amines may also be included in the gel as well as fillers, inhibitors, plasticizers, non reactive polymers, solvents and adhesion promoters.

By the term "gel," we mean a radiation-curable composition comprising photoinitiator, ethylenically unsaturated monomers and/or oligomers, having a viscosity suitable for coating natural or artificial nails, or forming artificial nails and extensions, as well as adorning such nails.

The aluminum platelets are generally dispersed in a pharmacologically acceptable solvent. This solution may contain viscosity modifiers to help maintain suspension of the platelets. Examples of inorganic based modifiers useful in the invention include but are not limited to: calcium, zinc or aluminum stearate, silica, fumed silica such as that available as Aerosil ® from Evonik Industries or Cab-O-Sil® available from Cabot Corporation, diatomaceous earth, bentonite clay, kaolinite, pyrophyllite, sericite, saponite, smectic/vermiculites (montmorillinite, beidillite, nontronite, hectorite and saponite), organic modified clays including but not limited to stearalkonium or distearalkonium bentonite and hectorite and others that are available from Elementis Specialties under the trade name of Bentone® and Garamites® from Rockwood Additives such as Garamite® 1958, talc, mica, zirconium oxide, zinc oxide and magnesium oxide. Organic based thixotropic additives may also be used provided that their molecular weight is low enough that they do not form effective films which interfere with the haze values obtained in the coating. A Molecular weight of less 1000 is preferred and less than 500 is most preferred. Examples of organic based thixotropic additives include but are not limited to: hydrogenated castor oils, hydrogenated castor oil waxes, inorganically modified castor oils, organically modified castor oils such as those sold by Elementis Specialties under the Thixcin® trademark, triglycerides such as glyceryl tri-12-hydroxy stearate, polyamides and modified polyamides such as 12-hydroxystearic acid diamide of ethylene diamine, 12-hydroxystearicacid diglycolamide, N-stearyl ricinoleamide, N-stearyl stearamide and other polyamide waxes. Included in these polyamide materials are those sold commercially by Kusumoto Chemicals Industries under the Disparlon® trademark, by Lehmann and Voss under the Luvotix® trademark, by Elementis Specialties under the Thixatrol® trademark, polyethylene oxide waxes, urea urethanes believed to be exemplified by those sold by Byk Incorporated as, for example, by Byk-410, Byk-411, and Byk-420, acrylic resins, amine salts of polymeric polyesters, salts of linear polyaminoamide and polymeric polyester, alkyl sulfonate, alkylallyl sulfonate, colloidal ester, polyester resin such as those sold by Elementis Specialties under the Thixatrol® trademark, phenol resin, melamine resin, epoxy resin, urethane resin, styrene butadiene polymers, polyimide resin, and polyester amides. Materials such as those sold by Byk under the trademarks of Anti-Terra® and Bykumen® can also be used.

### EXAMPLES

The following examples are presented to describe to the skilled artisan a few embodiments of the invention.

While the examples below are applied to a Colorpop, the same results were obtained when the laminate coatings were applied using color wheels such as those used for standard testing of nail coatings and on natural nails.

### Example 1 - Preparation of Laminate according to the Invention

To a Colorpop artificial nail available from Colorpops International was applied with a nail brush the formula given in Table 1 which can be used for base or top coats. The coating was cured for 2 minutes using a UV light designed for curing nail coatings and the tacky layer was removed by wiping with isopropanol. A layer of Shlenk Decomet 3014/10 aluminum platelets which had been diluted with butyl acetate to a 2% platelet level was then applied with a nail brush and the layer was allowed to dry for 30 seconds. A second layer of the formulation in Table 1 then applied and cured for 2 minutes using the same UV light followed by removal of the tacky layer with isopropanol. The resulting coating gave a superior mirror finish when compared to the laminate of Example 3.

**Table 1**

| **Material** | **Wt%** |
|---|---|
| Unsaturated Oligomer¹ | 69.19% |
| Trimethyol propane triacrylate | 13.42% |
| Hydroxy ethyl methacrylate | 13.37% |
| TPO² | 4.02% |

| | |
|---|---|
| ¹Urethane acrylate oligomer available from Sartomer Division of Arkema ²Diphenyl-(2,4,6-trimethylbenzoyl) phosphine oxide | |

### Example 2 (Invention)

The same procedure as used in Example 1 was used with the exception that the platelet dispersion contained 0.05% of Byk 410, a urea based thixotropic thickener. The resulting coating gave a superior mirror finish to the laminate of Example 3.

### Example 3 - Comparative

The same procedure as used in Example 1 was used with the exception that the platelet dispersion contained the following formulation given in Table 2.

**Table 2**

| **Material** | **Wt%** |
|---|---|
| Decomet 3104/10* | 4.08% |
| Butyl Acetate | 32.10% |
| Ethyl Acetate | 51.2% |
| Isopropyl Alcohol | 1.89% |
| Ethyl Alcohol | 5.53% |
| Dimethicone | 0.01% |
| Nitrocellulose 5/6 sec | 4.38% |
| Camphor | 0.8% |

| | |
|---|---|
| *As supplied - 10% dispersion in Ethyl Acetate | |

## Claims

1. A multi-layer cosmetic laminate for decorating natural or artificial human nails comprised of at least three layers, a UV cured coating as the first layer, a layer comprising aluminum platelets as the second layer, and another UV cured coating layer as the third layer, the second layer which comprises aluminum platelets being substantially free of film former.

2. The laminate of claim 1 comprised of one or more layers in addition to the first, second, and third layers, the one or more layers in addition being one or more enamel layers and/or one or more UV cured gel coat layers.

3. The laminate of claim 1 having a haze value greater than 950 HU.

4. The laminate of claim 1 wherein the UV cured coating layers are each formed by curing under UV radiation a composition comprising one or more ethylenically unsaturated monomers, one or more ethylenically unsaturated oligomers, or mixtures said ethylenically unsaturated monomer or monomers and said ethylenically unsaturated oligomer or oligomers, and a photoinitiator.

5. The laminate of claim 4 wherein monomers and/or oligomers are selected from mono-, di-, tri-, and tetra-functional ethylenically unsaturated monomers and/or oligomers.

6. The laminate of claim 4 wherein monomers and/or oligomers are (meth)acrylic.

7. The laminate of claim 4 wherein comprising oligomers which are aliphatic polyester based urethane diacrylate oligomers.

8. The laminate of claim 1 wherein the second layer comprising aluminum platelets is formed subsequent to curing the first base coat layer by coating the cured first base coat layer with a suspension of aluminum platelets in solvent and then evaporating the solvent to form a dry coating of aluminum.

9. The laminate of claim 8 wherein the solvent is selected from the group consisting of ethyl acetate, butyl acetate, acetone, and mixtures thereof.

10. The laminate of claim 1 wherein the third layer is free of pigment.

11. The laminate of claim 1 wherein a fourth layer is coated on top of the three layer laminate.

12. The laminate of claim 1 wherein second layer comprises a leafing agent.

13. The laminate of claim 1 wherein second layer comprises a leafing agent comprising a long chain phosphoric acid ester.

14. The laminate of claim 1 wherein aluminum platelets of the second layer are made by a method which comprises physical vapor deposition.

15. The laminate of claim 1 wherein aluminum platelets are multi-layered effect pigments created by physical vapor deposition having an aluminum core with MgF2 layers deposited thereon and a grating with a defined spacing between grating structures embossed on these low refracting layers which produces a prismatic rainbow-like diffraction effect when observed under visible light.

16. A method of decorating natural or artificial human nails comprising
a) applying a first layer comprising a UV curable gel coating composition comprising one or more ethylenically unsaturated monomers, one or more ethylenically unsaturated oligomers, or mixtures of said ethylenically unsaturated monomer or monomers and said ethylenically unsaturated oligomer or oligomers, and a photoinitiator;
b) curing the first layer under UV radiation;
c) applying a second layer comprising aluminum platelets which is substantially free of film former;
d) applying a third layer comprising the UV curable gel coating composition; and
e) curing the third layer under UV radiation.

## Patentansprüche

1. Vielschichtiges kosmetisches Laminat zum Dekorieren von natürlichen oder künstlichen menschlichen Nägeln, umfassend wenigstens drei Schichten, eine UV-gehärtete Beschichtung als die erste Schicht, eine Schicht, umfassend Aluminiumplättchen als die zweite Schicht, und eine weitere UV-gehärtete Beschichtungsschicht als die dritte Schicht, wobei die zweite Schicht, die Aluminiumplättchen umfasst, im Wesentlichen frei von filmbildender Substanz ist.

2. Laminat nach Anspruch 1, umfassend eine oder mehrere Schichten zusätzlich zu der ersten, zweiten und dritten Schicht, wobei die eine oder mehreren zusätzlichen Schichten eine oder mehrere Emaille-Schichten und/oder eine oder mehrere UV-gehärtete Gel-Beschichtungsschichten ist (sind).

3. Laminat nach Anspruch 1 mit einem Trübungswert größer als 950 HU.

4. Laminat nach Anspruch 1, wobei die UV-gehärteten Beschichtungsschichten jeweils gebildet werden durch Aushärten unter UV-Bestrahlung von einer Zusammensetzung, umfassend ein oder mehrere ethylenisch ungesättigte Monomere, ein oder mehrere ethylenisch ungesättigte Oligomere oder Mischungen des ethylenisch ungesättigten Monomers oder der Monomere und des ethylenisch ungesättigten Oligomers oder der Oligomere, und einen Fotoinitiator.

5. Laminat nach Anspruch 4, wobei die Monomere und/oder Oligomere ausgewählt sind aus mono-, di-, tri- und tetra-funktionellen ethylenisch ungesättigten Monomeren und/oder Oligomeren.

6. Laminat nach Anspruch 4, wobei Monomere und/oder Oligomere (Meth)acryl sind.

7. Laminat nach Anspruch 4, wobei es Monomere umfasst, die Urethan-Diacrylat-Oligomere auf aliphatischer Polyesterbasis sind.

8. Laminat nach Anspruch 1, wobei die zweite Schicht, umfassend Aluminiumplättchen, im Anschluss an das Aushärten der ersten Grund-Beschichtungsschicht gebildet wird durch Beschichten der ausgehärteten ersten Grund-Beschichtungsschicht mit einer Suspension von Aluminiumplättchen in Lösungsmittel und dann durch Verdampfen des Lösungsmittels, um eine trockene Aluminiumbeschichtung zu bilden.

9. Laminat nach Anspruch 8, wobei das Lösungsmittel ausgewählt ist aus der Gruppe, bestehend aus Ethylacetat, Butylacetat, Aceton und Mischungen davon.

10. Laminat nach Anspruch 1, wobei die dritte Schicht frei von Pigment ist.

11. Laminat nach Anspruch 1, wobei eine vierte Schicht auf dem dreischichtigen Laminat aufgetragen wird.

12. Laminat nach Anspruch 1, wobei die zweite Schicht ein Abblätterungsmittel umfasst.

13. Laminat nach Anspruch 1, wobei die zweite Schicht ein Abblätterungsmittel umfasst, umfassend einen langkettigen Phosphorsäureester.

14. Laminat nach Anspruch 1, wobei die Aluminiumplättchen der zweiten Schicht mittels eines Verfahrens gemacht werden, das eine physikalische Abscheidung aus der Dampfphase umfasst.

15. Laminat nach Anspruch 1, wobei die Aluminiumplättchen vielschichtige Effektpigmente sind, die mittels physikalischer Abscheidung aus der Dampfphase mit einem Aluminiumkern mit darauf abgelegten MgF₂-Schichten und einem Gitter mit einem definierten Zwischenraum zwischen den Gitterstrukturen erzeugt werden, die auf diesen niedrigbrechenden Schichten geprägt sind, was einen prismatischen regenbogenartigen Beugungseffekt bei Beobachtung unter sichtbarem Licht erzeugt.

16. Verfahren zum Dekorieren von natürlichen oder künstlichen menschlichen Nägeln, umfassend:
a) Auftragen einer ersten Schicht, umfassend eine UV-härtbare Gel-Beschichtungszusammensetzung, umfassend ein oder mehrere ethylenisch ungesättigte Monomere, ein oder mehrere ethylenisch ungesättigte Oligomere oder Mischungen des ethylenisch ungesättigten Monomers oder der Monomere und des ethylenisch ungesättigten Oligomers oder der Oligomere, und einen Fotoinitiator;
b) Aushärten der ersten Schicht unter UV-Bestrahlung;
c) Auftragen einer zweiten Schicht, umfassend Aluminiumplättchen, die im Wesentlichen frei von filmbildender Substanz ist;
d) Auftragen einer dritten Schicht, umfassend die UV-härtbare Gel-Beschichtungszusammensetzung; und
e) Aushärten der dritten Schicht unter UV-Bestrahlung.

## Revendications

1. Elément cosmétique stratifié multicouches destiné à la décoration d'ongles humains naturels ou artificiels constitué d'au moins trois couches, à savoir une couche de revêtement durcie aux UV en tant que première couche, une couche renfermant des plaquettes en aluminium en tant que seconde couche et une autre couche de revêtement durcie aux UV en tant que troisième couche, la seconde couche qui renferme des plaquettes d'aluminium étant essentiellement exempte de composés filmogènes.

2. Elément stratifié conforme à la revendication 1,
comprenant au moins une couche en plus de la première de la seconde et de la troisième couches, cette couche additionnelle étant constituée par une couche de laque et/ou au moins une couche de gel durcie aux UV.

3. Elément stratifié conforme à la revendication 1, ayant une valeur de trouble supérieure à 950 HU.

4. Elément stratifié conforme à la revendication 1,
dans lequel les couches de revêtement durcies aux UV sont chacune formées par polymérisation sous rayonnement UV d'une composition renfermant au moins un monomère à insaturation éthylénique, au moins un oligomère à insaturation éthylénique, ou des mélanges de tels monomères à insaturation éthylénique, et oligomères à insaturation éthylénique ainsi qu'un initiateur photochimique.

5. Elément stratifié conforme à la revendication 4,
dans lequel les monomères et/ou les oligomères sont choisis parmi des monomères et/ou oligomères à insaturation éthylénique mono-, di-, tri-, et tétra-fonctionnels.

6. Elément stratifié conforme à la revendication 4,
dans lequel les monomères et/ou les oligomères sont des monomères et/ou des oligomères (meth)acryliques.

7. Composé stratifié conforme à la revendication 4,
renfermant des oligomères constitués par des oligomères d'uréthane diacrylate à base de polyester aliphatique.

8. Composé stratifié conforme à la revendication 1,
dans lequel la seconde couche renfermant des plaquettes d'aluminium est formée après la polymérisation de la première couche de revêtement de base par revêtement de cette première couche avec une suspension de plaquettes d'aluminium dans un solvant puis évaporation du solvant pour former un revêtement d'aluminium sec.

9. Elément stratifié conforme à la revendication 8,
dans lequel le solvant est choisi dans le groupe formé par les solvants suivants :
acétate d'éthyle, acétate de butyle, acétone et leurs mélanges.

10. Elément stratifié conforme à la revendication 1,
dans lequel la troisième couche est exempte de pigments.

11. Elément stratifié conforme à la revendication 1,
dans lequel une quatrième couche est appliquée sur la troisième couche du composé stratifié.

12. Elément stratifié conforme à la revendication 1,
dans lequel la seconde couche renferme un agent de feuilletage.

13. Elément stratifié conforme à la revendication 1,
dans lequel la seconde couche comprend un agent de feuilletage renfermant un ester d'acide phosphorique à longue chaîne.

14. Elément stratifié conforme à la revendication 1,
dans lequel les plaquettes d'aluminium de la seconde couche sont obtenues par un procédé comprenant une étape de dépôt physique en phase vapeur.

15. Elément laminé conforme à la revendication 1,
dans lequel les plaquettes d'aluminium sont des pigments à effets multicouches obtenues par dépôt physique en phase vapeur ayant un noyau en aluminium sur lequel sont déposées des couches en MgF2 et un grillage avec un espacement défini entre des structures de grillage estampé sur ces couches faiblement réfractives produisant un effet de diffraction similaire à un arc-en-ciel prismatique lors d'une observation sous lumière visible.

16. Procédé de décoration d'ongles humains naturels ou artificiels comprenant des étapes consistant à :
a) appliquer une première couche comprenant une composition de revêtement sous forme de gel durcissable aux UV renfermant au moins un monomère à insaturation éthylénique, au moins un oligomère à insaturation éthylénique ou des mélanges de tels monomères à insaturation éthylénique et oligomères à insaturation éthylénique ainsi qu'un initiateur photochimique,
b) polymériser la première couche sous l'action d'un rayonnement UV,
c) appliquer une seconde couche renfermant des plaquettes d'aluminium qui est essentiellement exempte de composés filmogènes,
d) appliquer une troisième couche renfermant une composition de revêtement sous forme de gel durcissable aux UV, et
e) polymériser la troisième couche sous l'action d'un rayonnement UV.
